# EUROPEAN PATENT SPECIFICATION

(11) **EP 2 693 956 B1**
(45) Date of publication and mention of the grant of the patent: **10.06.2015**
(21) Application number: 11716344.4
(22) Date of filing: 07.04.2011
(51) Int. Cl.: A61B 17/16, B23B 31/00

(54) **CUTTING BURR SHANK CONFIGURATION**
BOHRERSCHAFT
TIGE DE FORET

(43) Date of publication of application: 12.02.2014
(73) Proprietor: DePuy Synthes Products, LLC, Raynham, MA 02767-0350 (US)
(72) Inventor: DEL RIO, Eddy H., Palm Beach Gardens, Florida 33410 (US); ENCK, Duane Jeffrey, Palm Beach Gardens, Florida 33410 (US)
(74) Representative: Jackson, Richard Eric
(86) International application number: PCT/US2011/031505
(87) International publication number: WO 2012/138337

(56) References cited:
- US-A- 5 037 251
- US-A1- 2002 009 341
- US-A1- 2002 159 850
- US-A1- 2003 060 829
- US-A1- 2003 060 841
- US-A1- 2006 049 587
- US-B1- 6 302 408

## Description

### BACKGROUND

When performing surgery, surgeons may utilize a surgical drilling instrument for drilling, cutting or shaping bones that utilize numerous different kinds and sizes of cutting burrs and attachments. During certain medical operations, the cutting burr needs to be changed. The change must be done timely and efficiently in view of the surgical demands. To this end, the portion of the cutting burr, namely, the proximate end of the shank typically lacks a configuration to accommodate this change of the cutting burr.

US 2002/0009341 A1 discloses a cutting burr according to the preamble of claim 1.

### SUMMARY

The present invention provides cutting burrs as recited in the claims.

Disclosed herein is a cutting burr that provides for a quick release that is fast and simple, and which facilitates the insertion of the cutting burr into a surgical drilling instrument. The first diamond-shaped portion may be formed at an edge of the proximal end of the cutting burr and provides a positive connection with a drive spindle that is connected to a drive motor of the surgical drilling instrument. The locking pawl may engage the second diamond-shaped portion to lock the cutting burr into the surgical drilling instrument with substantially no axial movement.

In some implementations, a detent pawl is provided to hold the cutting burr within the surgical instrument when it is in a loading position. The detent pawl may engage the axially disposed diamond-shaped portion at a side opposite the locking pawl.

In some implementations, the first diamond-shaped portion is sized such that it can be used with older surgical drilling instruments that may not be provided with a complementary receiving recess for the first diamond-shaped portion.

### BRIEF DESCRIPTION OF THE DRAWINGS

The foregoing summary is better understood when read in conjunction with the appended drawings. For the purposes of illustration, there is shown in the drawings exemplary implementations; however, these implementations are not limited to the specific methods and instrumentalities disclosed. In the drawings:
Fig. 1 a fragmentary top plan view illustrating the axially spaced six-sided diamond-shaped cut out portion or portions formed on the proximate end of the shank of the cutting burr;
Fig. 2 is a perspective view of Fig. 1;
Fig. 3 is another prospective view of Fig. 1 slightly turned illustrating one of the facets in each of the six-sided diamond-shaped portions;
Fig. 4 is another perspective view of Fig. 2 slightly turned illustrating the top facets of the six-sided diamond-shaped portions;
Fig. 5 is an end view taken along lines 5-5 of Fig. 3 illustrating the shape of the six-sided,diamond-shaped portion formed in the cutting burr shank;
Fig. 6 is a sectional view taken along lines 6-6 of Fig. 4 illustrating the shape of the six-sided diamond-shaped portion and illustrating the different sizes and the orientation of the six-sided diamond portion formed in the cutting burr shank;
Figs. 7A and 7B illustrate a backwards compatibility of the cutting burr of Figs. 1-6 within a receiving portion of conventional surgical drill;
Figs. 8A and 8B illustrate a self-alignment aspect of the diamond-shaped portion at a proximal end of the cutting burr in relation to a keyed slot of a surgical drill;
Fig. 9 is an elevated view of the cutting burr with a spherical shaped cutting bit illustrating the diamond-shaped portions formed in the shank thereof;
Fig. 10 is another elevated view of an example cutting burr; and
Fig. 11 is another elevated view of an example cutting burr.

### DETAILED DESCRIPTION

As used herein, the term "cutting burr" may be analogous with terms such as bit, drill bit, surgical drill bit and the like. The term "attachment" may have several meanings within the text of this application, but when generalized as a component of a surgical drilling instrument it refers to a portion of the instrument that attaches to the end of the motor/locking mechanism and receives the cutting burr. An "attachment" may be a controlled depth attachment, a minimally invasive attachment and the like. The surgical drilling instrument may include an integral motor (electric or pneumatic) and a locking mechanism and an attachment releasably connected to the locking mechanism.

High speed surgical drills are increasingly being used by surgeons when performing delicate bone dissection in areas such as the cervical and lumbar spine. Such surgical drills operate at very high R.P.M., and are able to rotationally drive multiple types of attachments and cutting burrs. As will be described below, a cutting burr of the present disclosure includes a shank that defines two substantially diamond-shaped portions. The substantially diamond-shaped portions provide for ease of insertion and removal of the cutting burr to and from a compatible surgical drill. The substantially diamond-shaped portions also enable the surgical drill to direct higher levels of torque to the cutting burr during surgical procedures.

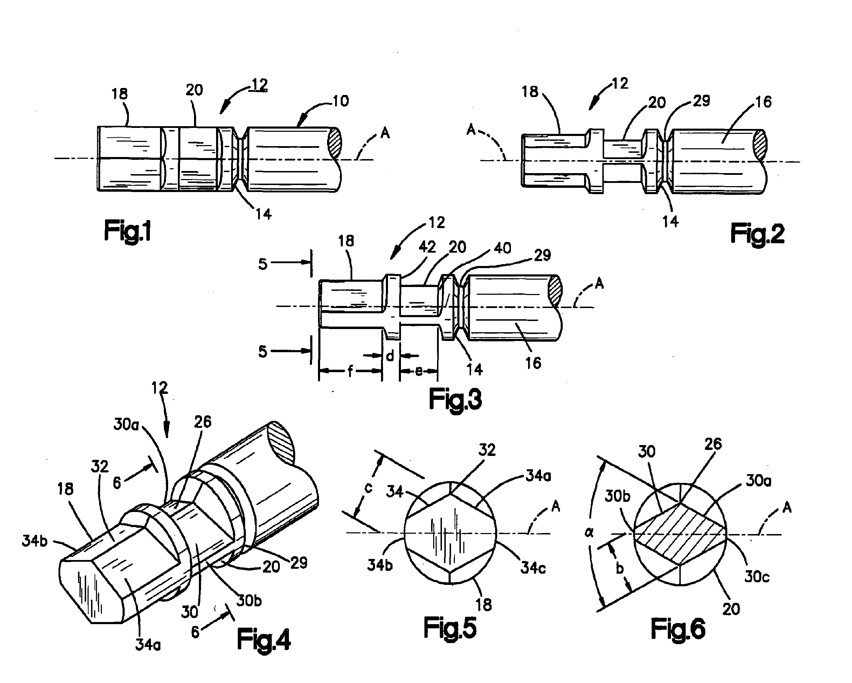

Referring to Figs. 1-6, the cutting burr is generally illustrated by reference numeral 10. The attachment portion 12 of the shank 16 of the cutting burr 10 is generally shown as reference numeral 12. A proximal end 14 of the shank 16 is formed with a pair of axially spaced six-sided diamond-shaped portions 18 and 20. As shown in Figs. 4 and 5, an upper surface of portion 18 includes an apex 32 and a pair of facets 34 and 34a also fairing to side edges 34b and 34c. The side edges 34b and 34c are curved to match the radius of curvature of an outer surface of the shank 16. As shown in Figs. 4 and 6, an upper surface 24 of the portion 20 includes apex 26 and a pair of facets 30 and 30a fairing from the apex 26 to the side edges 30b and 30c. The side edges 30b and 30c are curved to match the radius of curvature of an outer surface of the shank 16.

As shown in the Figs. the diametrical dimensions of the vertices in both portions is less than the diameter of the main body of the shank. The shank 16 may include an annular groove 29. The lower surfaces of the pair of six-sided diamond portions 18 and 20 are a mirror image of the upper surface. While the diamond-shaped portions 18 and 20 are described as being "diamond-shaped," it is noted that such terminology is intended to encompass any six-sided (hexagon) shape having a cross-section with flat edges that meet at a six vertices, curved edges that meet at six points, or some combination of both to form the six sides. The flat and curved edges, and combinations thereof, may be applied to other polygon shapes having different numbers of sides.

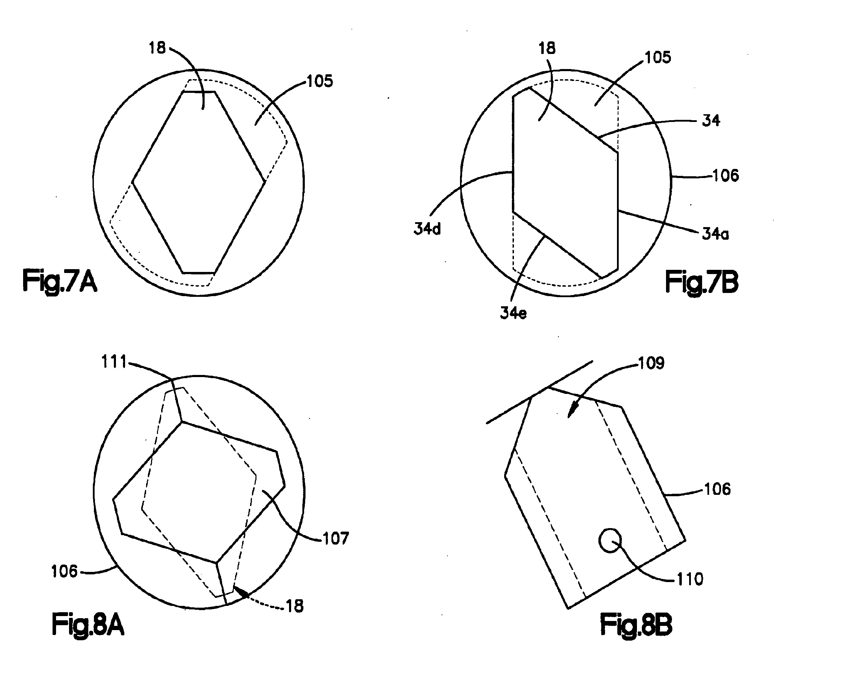

The diamond-shaped portion 18 at the outermost proximal end is designed to be inserted into a mating drive portion of a surgical drill, as will be described with reference to Figs. 8A and 8B. The diamond-shaped portion 20 is provided as an abutment surface of a retractable locking pawl of the surgical drill to provide axial locking of the shank 16 within the surgical drill. The locking pawl may axially abut the adjacent abutment surface of the diamond-shaped portion 20 to axially lock the cutting burr 10 in place, thus providing substantially zero axial movement. For example, an engagement portion of locking pawl may be contoured having a generally V-shape with inner surfaces that fit against the facets 30 and 30a of the diamond-shaped portion 20.

As shown in Fig. 3, a back wall 42 may be formed perpendicular with relation to the central line A and faces a front wall 40 that is tapered from the facet (e.g., 30a) to the outside diameter of the shank 16. In accordance with some aspects, an engagement face of the locking pawl may abut against the back wall 42 to provide axial locking of the cutting burr 10 within the surgical drill. A tapered front wall 40 may facilitate the engagement of the locking pawl into the diamond-shaped portion 20.

The diamond-shaped portion 20 may also be engaged by a detent pawl of the surgical drill. For example, an engagement end of detent pawl may be contoured, e.g., having a generally hill shape to partially fit into the diamond-shaped portion 20 on an opposite side of the engagement end of the locking pawl. The detent pawl may be provided to apply a sufficient force on the diamond-shaped portion 20 to allow the cutting burr 10 to be moved in and out of the surgical drill, while reducing the likelihood that the cutting burr will inadvertently fall out of the surgical drill when in a loading position.

As shown by the a comparison of the sectional views of the diamond-shaped portions 18 and 20 (Figs. 5 and 6), the two diamond shapes may be different in size, where the diamond shape in diamond-shaped portion 18 is larger than the diamond shape of the diamond-shaped portion 20. As illustrated, the vertices 32 and 36 fall below the outer diameter of the shank 16 and both diamond shapes are in axial alignment with each other and may be oriented in parallel relationship. In some implementations, the diamond-shaped portion 20 and the diamond-shaped portion 18 may be the same size, or the diamond-shaped portion 18 may be larger than the diamond-shaped portion 20. In the various configurations, the vertices 26 and 32 of diamond-shaped portions 20 and 18, respectively, are along a same line and in a same plane as the center line A. Exemplary dimensions of the six-sided diamond diamond-shaped portions 18 and 20 are listed in degrees (°) and millimetres (mm) (inches (")) and may be substantially as follows:
The angle of the facets of the six-sided diamond in the diamond-shaped portion 20 - a = 47°;
The width of the facets of the six-sided diamond in the diamond-shaped portion 20 - b = 1.168mm (0.046");
The width of the facets of the six-sided diamond in the diamond-shaped portion 18 - c = 1.651 mm (0.065");
The width of the shank 16 at the space between diamond-shaped portions 18 and 20 - d = 0.737mm (0.029");
The length of the diamond-shaped portion 20 - e = 1.727mm (0.068"); and
   The length between the proximal end and the back wall of diamond-shaped portion 18 - f = 3.785mm (0.149"). This dimension may contribute to the feature of substantially reducing the axial play of the cutting burr.

Thus, in accordance with the above, the diamond-shaped portions 18 and 20 provide sufficient cross-sectional dimensions to meet strength and reliability requirements needed for high-speed, large force surgical applications. Facets 34 and 34a of the diamond shape 18 provide positive engagement surfaces in both clockwise and counter-clockwise rotational directions and are sufficiently sized to withstand rotations forces in either direction without wobbling within the surgical drill. For example, some surgical drills provide bi-directional rotation, allowing the surgeon to selectively reverse rotation for various surgical techniques. In conventional designs, there may be rotational play between a bit end and a drive portion. However, the symmetrical diamond facets 34 and 34a of the diamond-shaped portion 18 provide substantial drive surfaces in either direction.

With reference to Figs. 7A and 7B, the diamond-shaped portion 18 at the outermost proximal end of the cutting burr 10 is designed to have unidirectional backward compatibility with older drill instruments in accordance with aspects of the disclosure. For example, a conventional drill instrument may include an insert 106 that defines a generally rectangular slot 105 having rounded side walls. The rounded side walls may be shaped with a radius of curvature that parallels the outer wall of the insert 106. Conventional cutting burrs may include a complementary generally rectangular portion having rounded side walls that is received by the slot 105. The insert 106 may be driven by a motor, thus providing rotational force on the cutting burr.

As shown in Fig. 7A, in accordance with some implementations, facets 34a and 34d of the diamond-shaped portion 18 engage the inner walls of the slot 105. The dimension c of the diamond-shaped portion 18, noted above, may be sized such that the surface area of the facets 34a and 34d is substantial enough to withstand the torque provided by the motor of the conventional drill instrument. Thus, the cutting burr 10 of the present disclosure may be utilized by conventional drill instruments.

Referring now to Figs. 8A and 8B, in some implementations, the cutting burr 10 of the present disclosure provides for a level of self-alignment within the insert 106. The insert 106 may be provided in a compatible surgical drill and define a diamond-shaped key slot 107, a pointed shaped inlet end 109, and opposing holes 110 that formed in the insert 106 for receiving dowel pin which may serve to locate the cutting burr 10 when inserted into the key slot 107. The inlet end 109 serves to facilitate the alignment and insertion of the cutting burr 10 as it is advanced toward and into the key slot 107 of the insert 106. For example, if the diamond-shaped portion 18 is not in alignment with the key slot 107 (Fig. 8A), a bottom surface of the diamond-shaped portion 18 will contact an apex 111 of the inlet end 109 causing the cutting burr 10 to rotate into alignment with the key slot 107. As such, the cooperative engagement of the diamond-shaped portion 18 and inlet end 109 facilitates the easy insertion of the cutting burr 10 into the compatible surgical drill. As such, the diamond portion 18 serves to provide a secure connection in the key slot 107.

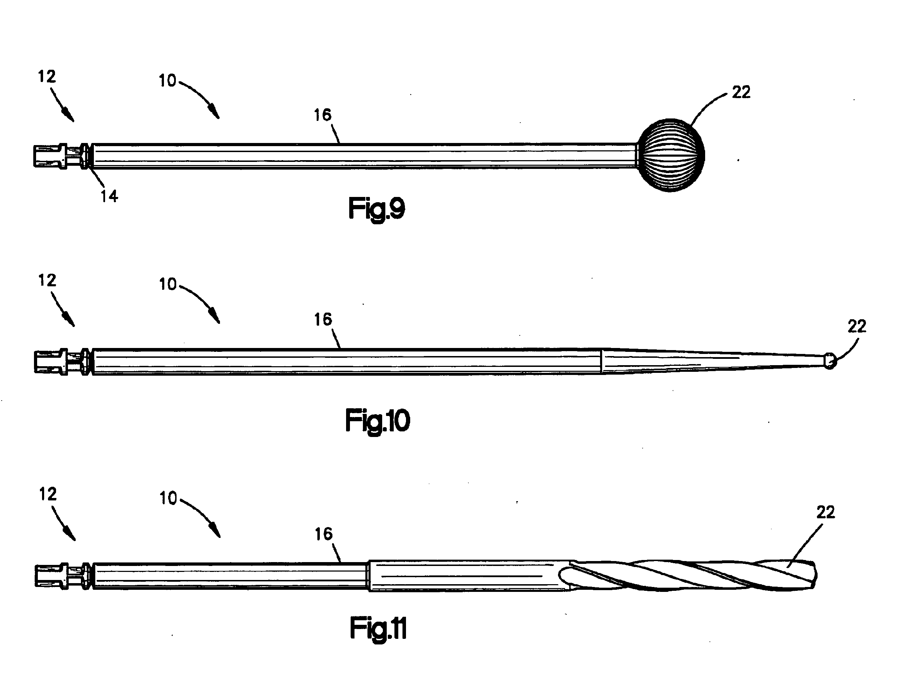

Figs. 9, 10, and 11 illustrate different example cutting bits 22 provided at a distal end on the shank 16. As described above, the shank 16 may include the attachment portion 12. The cutting bits 22 may be milled or cut-out portions. The cutting burr 10 in Fig. 9 exemplifies a fluted ball or drill bit; the cutting burr 10 in Fig. 10 exemplifies a diamond ball; and the cutting burr 10 in Fig. 11 exemplifies a twist drill. The cutting bits 22 are presented only as examples and are not intended to limit the scope of the present disclosure as numerous variations are possible.

Thus, as described above, a cutting burr is provided with an attachment end that has a configuration and dimensions that serve to facilitate the insertion of the cutting burr into the surgical cutting instrument. When locked in the running position there is a structure that prevents the cutting burr from having any axial movement. Also, there is a positive connection such that the cutting burr rotates concentrically without any wobbling motion.

## Claims

1. A cutting burr (10) having a shank (16) with an engagement end at a proximal end (14) of the shank (16) adapted to be received by a surgical drill, comprising:
a first six-sided, diamond-shaped portion (18) formed at the proximal end of the shank and having first side edges (34b, 34c), the first diamond-shaped portion having a first pair of facets (34, 34a) that meet at a first apex (32) and an opposing second pair of facets; and
a second six-sided, diamond-shaped portion (20) axially spaced along a shaft of the shank (16) from the first diamond-shaped portion (18) and having second side edges (30b, 30c), the second diamond-shaped portion (20) having a third pair of facets (30, 30a) that meet at a second apex (26) and an opposing fourth pair of facets,
wherein the first diamond-shaped portion (18) is adapted to be inserted into a complementary shaped mating recess of the surgical drill, and
wherein the second diamond-shaped portion (20) is adapted to mate with a complementary locking pawl of the surgical drill, **characterized in that**:
the first side edges (34b, 34c) and second side edges (30b, 30c) are arched having a radius of curvature of an outside diameter of the shank (16).

2. The cutting burr (10) of claim 1, wherein each of the facets has a substantially flat surface.

3. The cutting burr (10) of claim 1, wherein the first pair of facets (34, 34a) and the second pair of facets are symmetrical about a center plane of the shank (16).

4. The cutting burr (10) of claim 1, wherein diametrical dimensions of vertices of the first diamond-shaped portion (18) and the second diamond-shaped portion (20) is less than a diameter of the shank.

5. The cutting burr (10) of claim 1, wherein the shank (16) includes an annular groove (29).

6. The cutting burr (10) of claim 1, wherein the third pair of facets (30, 30a) are formed at a predetermined angle for receiving the locking pawl having a complementary shape to that of the third pair of facets (30, 30a).

7. The cutting burr (10) of claim 6, wherein the complementary shape is generally a V-shape.

8. The cutting burr (10) of claim 1, wherein the fourth pair of facets of the second diamond-shaped portion (20) are adapted to be engaged by a detent pawl of the surgical drill, wherein the detent pawl is generally hill-shaped to partially fit into the second diamond-shaped portion (20) on an opposite side of the locking pawl.

9. The cutting burr (10) of claim 1, wherein the first diamond-shaped portion (18) is adapted to accept rotational engagement forces of the surgical drill from the mating recess, and wherein the second diamond-shaped portion (20) in cooperation with the locking pawl can provide for axial locking of the cutting burr (10).

10. The cutting burr (10) of claim 9, wherein a back wall (42) of the second diamond-shaped portion (20) is perpendicular to a center line of the shank (16) and faces a front wall (40) of the second diamond-shaped portion (20) that is tapered from each facet to an outside surface of the shank (16).

11. The cutting burr (10) of claim 10, wherein the back wall (42) is adapted to be engaged by an engagement end of the locking pawl.

12. The cutting burr (10) of claim 10, wherein the tapered front wall (40) can facilitate the engagement of the locking pawl into the second diamond-shaped portion (20).

13. The cutting burr (10) of claim 9, wherein the first pair of facets (34, 34a) and the second pair of facets are able to provide positive engagement surfaces within the mating recess in both clockwise and counter-clockwise rotational directions of the surgical drill.

14. The cutting burr (10) of claim 1, wherein the first diamond-shaped portion (18) and the second diamond-shaped portion (20) are different sizes.

15. The cutting burr (10) of claim 14, wherein a width of each of the facets of the second diamond-shaped portion (20) is approximately 1.168mm (0.046"), wherein the third pair of facets and the fourth pair of facets are formed at approximately an angle of 47°, and wherein a length of the second diamond-shaped portion (20) is approximately 1.727mm (0.068").

16. The cutting burr (10) of claim 14, wherein a width of each of the facets of the first diamond-shaped portion (18) is approximately 1.651mm (0.065"), and wherein a length between the proximal end and a back wall of first diamond-shaped portion (18) is approximately 3.785mm (0.149").

17. The cutting burr (10) of claim 14, wherein a width of an axial space between the first diamond-shaped portion (18) and the second diamond-shaped portion (20) is approximately 0.737mm (0.029").

18. The cutting burr (10) of claim 1, wherein the first diamond-shaped portion (18) and the second diamond-shaped portion (20) are in axial alignment and are oriented in a parallel relationship.

19. The cutting burr (10) of claim 18, wherein the first apex (32) and the second apex (26) are in axial alignment.

20. The cutting burr (10) of claim 1, wherein the second diamond-shaped portion (20) defines a recess for axially locking the cutting burr (10) within a surgical drill.

21. The cutting burr (10) of claim 1, wherein a width of each of the facets of the first diamond-shaped portion (18) is sized to provide unidirectional backward compatibility with other surgical drills having different shaped mating recesses than the complementary shaped mating recess.

22. The cutting burr (10) of claim 1, wherein the complementary shaped mating recess is formed within an insert, the insert further defining a pointed shaped inlet end, and wherein the inlet end facilitates an alignment and insertion of the cutting burr (10) as it is advanced toward and into the mating recess.

23. The cutting burr (10) of claim 1, wherein the respective facets of the first and second six-sided portions (18, 20) are formed in parallel planes and are substantially flat.

24. The cutting burr (10) of claim 1, wherein the first and second apexes (32, 26) are disposed below a surface of the shank (16).

25. The cutting burr (10) of claim 1, wherein:
the shank (16) is cylindrically shaped;
a cutting bit (22) is formed at a distal end of the cutting burr (10); and
the second diamond-shaped portion (20) is a predetermined distance from the first diamond-shaped portion (18),
wherein the first and second apexes (32, 26) are axially aligned and the respective facets are disposed within parallel planes,
wherein the first diamond-shaped portion (18) is adapted to receive clockwise or counter-clockwise rotation forces applied by the surgical drill having a complementary keyed slot to receive the first diamond-shaped portion (18), and
wherein the second diamond-shaped portion (20) is adapted to be axially locked within the surgical drill.

26. The cutting burr (10) of claim 25, wherein the second diamond-shaped portion (20) can be engaged by a detent pawl of the surgical drill to hold the cutting burr (10) in place when the surgical drill is in a loading position.

## Patentansprüche

1. Bohrer (10) mit einem Schaft (16) mit einem Eingriffsende an einem proximalen Ende (14) des Schafts (16), das ausgelegt ist, von einem chirurgischen Bohrer aufgenommen zu werden, umfassend:
einen ersten sechsseitigen diamantförmigen Abschnitt (18), der an dem proximalen Ende des Schafts geformt ist und erste Seitenränder (34b, 34c) aufweist, wobei der erste diamantförmige Abschnitt ein erstes Paar von Facetten (34, 34a), die sich an einer ersten Spitze (32) treffen, und ein zweites gegenüberliegendes Paar von Facetten aufweist; und
einen zweiten sechsseitigen diamantförmigen Abschnitt (20), der entlang einer Achse des Schafts (16) im axialen Abstand von dem ersten diamantförmigen Abschnitt (18) angeordnet ist und zweite Seitenränder (30b, 30c) aufweist, wobei der zweite diamantförmige Abschnitt (20) ein drittes Paar von Facetten (30, 30a), die sich an einer zweiten Spitze (26) treffen, und ein gegenüberliegendes viertes Paar von Facetten aufweist,
worin der erste diamantförmige Abschnitt (18) zur Einführung in eine komplementär geformte passende Aussparung des chirurgischen Bohrers ausgelegt ist, und
worin der zweite diamantförmige Abschnitt (20) ausgelegt ist, mit einer komplementären Raste des chirurgischen Bohrers zusammenzupassen, **dadurch gekennzeichnet, dass**:
die ersten Seitenränder (34b, 34c) und die zweiten Seitenränder (30b, 30c) gebogen sind und einen Krümmungsradius eines Außendurchmessers des Schafts (16) aufweisen.

2. Bohrer (10) nach Anspruch 1, worin jede der Facetten eine im Wesentlichen flache Oberfläche aufweist.

3. Bohrer (10) nach Anspruch 1, worin das erste Paar von Facetten (34, 34a) und das zweite Paar von Facetten um eine Mittelebene des Schafts (16) symmetrisch sind.

4. Bohrer (10) nach Anspruch 1, worin diametrale Abmessungen von Scheitelpunkten des ersten diamantförmigen Abschnitts (18) und des zweiten diamantförmigen Abschnitts (20) kleiner als ein Durchmesser des Schafts sind.

5. Bohrer (10) nach Anspruch 1, worin der Schaft (16) eine ringförmige Nut (29) aufweist.

6. Bohrer (10) nach Anspruch 1, worin das dritte Paar von Facetten (30, 30a) zur Aufnahme der Raste, die eine zu der Gestalt des dritten Paars von Facetten (30, 30a) komplementäre Gestalt aufweist, in einem vorbestimmten Winkel geformt ist.

7. Bohrer (10) nach Anspruch 6, worin die komplementäre Gestalt allgemein V-förmig ist.

8. Bohrer (10) nach Anspruch 1, worin das vierte Paar von Facetten des zweiten diamantförmigen Abschnitts (20) für Eingriff durch eine Sperrklinke des chirurgischen Bohrers ausgelegt ist, worin die Sperrklinke allgemein hügelförmig ist, um teilweise in den zweiten diamantförmigen Abschnitt (20) auf einer gegenüberliegenden Seite der Raste zu passen.

9. Bohrer (10) nach Anspruch 1, worin der erste diamantförmige Abschnitt (18) ausgelegt ist, Dreheingriffskräfte des chirurgischen Bohrers von der passenden Aussparung anzunehmen, und worin der zweite diamantförmige Abschnitt (20) in Zusammenarbeit mit der Raste axiale Arretierung des Bohrers (10) ermöglichen kann.

10. Bohrer (10) nach Anspruch 9, worin eine Rückwand (42) des zweiten diamantförmigen Abschnitts (20) senkrecht zu einer Mittellinie des Schafts (16) ist und zu einer Vorderwand (40) des zweiten diamantförmigen Abschnitts (20) weist, die von jeder Facette zu einer Außenseite des Schafts (16) verjüngt ist.

11. Bohrer (10) nach Anspruch 10, worin die Rückwand (42) zum Eingriff durch ein Eingriffsende der Raste ausgelegt ist.

12. Bohrer (10) nach Anspruch 10, worin die verjüngte Vorderwand (40) den Eingriff der Raste in dem zweiten diamantförmigen Abschnitt (20) erleichtern kann.

13. Bohrer (10) nach Anspruch 9, worin das erste Paar von Facetten (34, 34a) und das zweite Paar von Facetten in Richtungen des chirurgischen Bohrers sowohl im Uhrzeigersinn als auch gegen den Uhrzeigersinn formschlüssige Eingriffsflächen in der passenden Aussparung bereitstellen können.

14. Bohrer (10) nach Anspruch 1, worin der erste diamantförmige Abschnitt (18) und der zweite diamantförmige Abschnitt (20) unterschiedliche Größen aufweisen.

15. Bohrer (10) nach Anspruch 14, worin eine Breite jeder der Facetten des zweiten diamantförmigen Abschnitts (20) ungefähr 1,168 mm (0,046 Zoll) beträgt, worin das dritte Paar von Facetten und das vierte Paar von Facetten in einem Winkel von ungefähr 47° geformt sind, und worin eine Länge des zweiten diamantförmigen Abschnitts (20) ungefähr 1,727 mm (0,068 Zoll) beträgt.

16. Bohrer (10) nach Anspruch 14, worin eine Breite jeder der Facetten des ersten diamantförmigen Abschnitts (18) ungefähr 1,651 mm (0,065 Zoll) beträgt, und worin eine Länge zwischen dem proximalen Ende und einer Rückwand des ersten diamantförmigen Abschnitts (18) ungefähr 3,785 mm (0,149 Zoll) beträgt.

17. Bohrer (10) nach Anspruch 14, worin eine Breite eines axialen Zwischenraums zwischen dem ersten diamantförmigen Abschnitt (18) und dem zweiten diamantförmigen Abschnitt (20) ungefähr 0,737 mm (0,029 Zoll) beträgt.

18. Bohrer (10) nach Anspruch 1, worin der erste diamantförmige Abschnitt (18) und der zweite diamantförmige Abschnitt (20) axial ausgerichtet sind und in einer parallelen Beziehung orientiert sind.

19. Bohrer (10) nach Anspruch 18, worin die erste Spitze (32) und die zweite Spitze (26) axial ausgerichtet sind.

20. Bohrer (10) nach Anspruch 1, worin der zweite diamantförmige Abschnitt (20) eine Aussparung zur axialen Arretierung des Bohrers (10) in einem chirurgischen Bohrer definiert.

21. Bohrer (10) nach Anspruch 1, worin eine Breite jeder der Facetten des ersten diamantförmigen Abschnitts (18) für unidirektionale Rückwärtskompatibilität mit anderen chirurgischen Bohrern, die anders geformte passende Aussparungen als die komplementär geformte passende Aussparung aufweisen, bemessen ist.

22. Bohrer (10) nach Anspruch 1, worin die komplementär geformte passende Aussparung in einem Einsatz geformt ist, wobei der Einsatz ferner ein spitz zulaufendes Einlassende definiert, und worin das Einlassende eine Ausrichtung und Einführung des Bohrers (10) erleichtert, wenn dieser zu und in die passende Aussparung vorgeschoben wird.

23. Bohrer (10) nach Anspruch 1, worin die jeweiligen Facetten der ersten und zweiten sechsseitigen Abschnitte (18, 20) in parallelen Ebenen geformt sind und im Wesentlichen flach sind.

24. Bohrer (10) nach Anspruch 1, worin die ersten und zweiten Spitzen (32, 26) unter einer Oberfläche des Schafts (16) angeordnet sind.

25. Bohrer (10) nach Anspruch 1, worin:
der Schaft (16) zylinderförmig ist;
eine Bohrschneide (22) an einem distalen Ende des Bohrers (10) geformt ist; und
der zweite diamantförmige Abschnitt (20) in einem vorbestimmten Abstand von dem ersten diamantförmigen Abschnitt (18) angeordnet ist,
worin die ersten und zweiten Spitzen (32, 26) axial ausgerichtet sind und die jeweiligen Facetten in parallelen Ebenen angeordnet sind,
worin der erste diamantförmige Abschnitt (18) zur Aufnahme von Drehkräften ausgelegt ist, die von dem chirurgischen Bohrer, der einen komplementären formschlüssigen Schlitz zur Aufnahme des ersten diamantförmigen Abschnitts (18) aufweist, im Uhrzeigersinn oder gegen den Uhrzeigersinn aufgebracht werden, und
worin der zweite diamantförmige Abschnitt (20) ausgelegt ist, axial in dem chirurgischen Bohrer arretiert zu werden.

26. Bohrer (10) nach Anspruch 25, worin eine Sperrklinke des chirurgischen Bohrers in den zweiten diamantförmigen Abschnitt (20) eingreifen kann, um den Bohrer (10) festzuhalten, wenn sich der chirurgische Bohrer in einer Ladestellung befindet.

## Revendications

1. Foret (10) ayant une tige (16) avec une extrémité d'engagement à une extrémité proximale (14) de la tige (16), prévu pour être reçu par une fraise chirurgicale, comprenant :
une première portion en forme de losange à six côtés (18) formée à l'extrémité proximale de la tige et ayant des premiers bords latéraux (34b, 34c), la première portion en forme de losange ayant une première paire de facettes (34, 34a) qui se rejoignent au niveau d'un premier sommet (32) et une deuxième paire de facettes opposée ; et
une deuxième portion en forme de losange à six côtés (20) espacée axialement le long d'un axe de la tige (16) depuis la première portion en forme de losange (18) et ayant des deuxièmes bords latéraux (30b, 30c), la deuxième portion en forme de losange (20) ayant une troisième paire de facettes (30, 30a) qui se rejoignent au niveau d'un deuxième sommet (26) et une quatrième paire de facettes opposée,
la première portion en forme de losange (18) étant prévue pour être insérée dans un retrait d'accouplement de forme complémentaire de la fraise chirurgicale, et
la deuxième portion en forme de losange (20) étant prévue pour s'accoupler avec un cliquet de verrouillage complémentaire de la fraise chirurgicale, **caractérisé en ce que** :
les premiers bords latéraux (34b, 34c) et les deuxièmes bords latéraux (30b, 30c) sont arqués avec un rayon de courbure d'un diamètre extérieur de la tige (16).

2. Foret (10) selon la revendication 1, dans lequel chacune des facettes a une surface substantiellement plate.

3. Foret (10) selon la revendication 1, dans lequel la première paire de facettes (34, 34a) et la deuxième paire de facettes sont symétriques par rapport à un plan central de la tige (16).

4. Foret (10) selon la revendication 1, dans lequel les dimensions diamétrales des sommets de la première portion en forme de losange (18) et de la deuxième portion en forme de losange (20) sont inférieures à un diamètre de la tige.

5. Foret (10) selon la revendication 1, dans lequel la tige (16) comporte une gorge annulaire (29).

6. Foret (10) selon la revendication 1, dans lequel la troisième paire de facettes (30, 30a) est formée suivant un angle prédéterminé pour recevoir le cliquet de verrouillage ayant une forme complémentaire à celle de la troisième paire de facettes (30, 30a).

7. Foret (10) selon la revendication 6, dans lequel la forme complémentaire est généralement une forme en V.

8. Foret (10) selon la revendication 1, dans lequel la quatrième paire de facettes de la deuxième portion en forme de losange (20) est prévue pour être amenée en prise avec un cliquet d'arrêt de la fraise chirurgicale, le cliquet d'arrêt étant généralement en forme de colline de manière à s'ajuster en partie à l'intérieur de la deuxième portion en forme de losange (20) sur un côté opposé du cliquet de verrouillage.

9. Foret (10) selon la revendication 1, dans lequel la première portion en forme de losange (18) est prévue pour accepter des forces d'engagement rotationnelles de la fraise chirurgicale depuis le retrait d'accouplement, et dans lequel la deuxième portion en forme de losange (20), en coopération avec le cliquet de verrouillage, peut assurer un verrouillage axial du foret (10).

10. Foret (10) selon la revendication 9, dans lequel une paroi arrière (42) de la deuxième portion en forme de losange (20) est perpendiculaire à un axe central de la tige (16) et est en regard d'une paroi avant (40) de la deuxième portion en forme de losange (20) qui est effilée à partir de chaque facette jusqu'à une surface extérieure de la tige (16).

11. Foret (10) selon la revendication 10, dans lequel la paroi arrière (42) est prévue pour être amenée en prise avec une extrémité d'engagement du cliquet de verrouillage.

12. Foret (10) selon la revendication 10, dans lequel la paroi avant effilée (40) peut faciliter l'engagement du cliquet de verrouillage dans la deuxième portion en forme de losange (20).

13. Foret (10) selon la revendication 9, dans lequel la première paire de facettes (34, 34a) et la deuxième paire de facettes sont en mesure de fournir des surfaces d'engagement positif à l'intérieur du retrait d'accouplement dans des directions rotationnelles de la fraise chirurgicale dans le sens horaire et dans le sens inverse.

14. Foret (10) selon la revendication 1, dans lequel la première portion en forme de losange (18) et la deuxième portion en forme de losange (20) ont des tailles différentes.

15. Foret (10) selon la revendication 14, dans lequel une largeur de chacune des facettes de la deuxième portion en forme de losange (20) mesure approximativement 1,168 mm (0,046"), la troisième paire de facettes et la quatrième paire de facettes étant formées suivant un angle d'environ 47°, et une longueur de la deuxième portion en forme de losange (20) étant d'environ 1,727 mm (0, 068").

16. Foret (10) selon la revendication 14, dans lequel une largeur de chacune des facettes de la première portion en forme de losange (18) mesure approximativement 1,651 mm (0,065"), et dans lequel une longueur entre l'extrémité proximale et une paroi arrière de la première portion en forme de losange (18) mesure approximativement 3,785 mm (0, 149") .

17. Foret (10) selon la revendication 14, dans lequel une largeur d'un espace axial entre la première portion en forme de losange (18) et la deuxième portion en forme de losange (20) mesure approximativement 0,737 mm (0,029").

18. Foret (10) selon la revendication 1, dans lequel la première portion en forme de losange (18) et la deuxième portion en forme de losange (20) sont alignées axialement et sont orientées suivant une relation parallèle.

19. Foret (10) selon la revendication 18, dans lequel le premier sommet (32) et le deuxième sommet (26) sont alignés axialement.

20. Foret (10) selon la revendication 1, dans lequel la deuxième portion en forme de losange (20) définit un retrait pour verrouiller axialement le foret (10) à l'intérieur d'une fraise chirurgicale.

21. Foret (10) selon la revendication 1, dans lequel une largeur de chacune des facettes de la première portion en forme de losange (18) est dimensionnée de manière à fournir une compatibilité vers l'arrière unidirectionnelle avec d'autres fraises chirurgicales ayant des retraits d'accouplement de formes différentes du retrait d'accouplement de forme complémentaire.

22. Foret (10) selon la revendication 1, dans lequel le retrait d'accouplement de forme complémentaire est formé à l'intérieur d'un insert, l'insert définissant en outre une extrémité d'entrée de forme pointue, et dans lequel l'extrémité d'entrée facilite l'alignement et l'insertion du foret (10) à mesure qu'il est avancé vers et dans le retrait d'accouplement.

23. Foret (10) selon la revendication 1, dans lequel les facettes respectives des première et deuxième portions à six côtés (18, 20) sont formées dans des plans parallèles et sont substantiellement plates.

24. Foret (10) selon la revendication 1, dans lequel les premier et deuxième sommets (32, 26) sont disposés sous une surface de la tige (16).

25. Foret (10) selon la revendication 1, dans lequel :
la tige (16) a une forme cylindrique ;
une partie de coupe (22) est formée à une extrémité distale du foret (10) ; et
la deuxième portion en forme de losange (20) est située à une distance prédéterminée de la première portion en forme de losange (18),
les premier et deuxième sommets (32, 26) étant alignés axialement et les facettes respectives étant disposées à l'intérieur de plans parallèles,
la première portion en forme de losange (18) étant prévue pour recevoir des forces de rotation dans le sens horaire ou dans le sens inverse, appliquées par la fraise chirurgicale, ayant une fente clavetée complémentaire pour recevoir la première portion en forme de losange (18), et
la deuxième portion en forme de losange (20) étant prévue pour être verrouillée axialement à l'intérieur de la fraise chirurgicale.

26. Foret (10) selon la revendication 25, dans lequel la deuxième portion en forme de losange (20) peut être amenée en prise avec un cliquet d'arrêt de la fraise chirurgicale pour retenir le foret (10) en position lorsque la fraise chirurgicale est dans une position de chargement.
